# EUROPEAN PATENT SPECIFICATION

(11) **EP 0 948 601 B1**
(45) Date of publication and mention of the grant of the patent: **14.02.2007**
(21) Application number: 97950805.8
(22) Date of filing: 11.12.1997
(51) Int. Cl.: C12N 7/02, C12N 7/00

(54) **METHODS FOR PURIFYING VIRUSES**
METHODEN ZUR VIRUS-REINIGUNG
PROCEDES POUR PURIFIER DES VIRUS

(30) Priority: 13.12.1996 US 766835
(43) Date of publication of application: 13.10.1999
(60) Divisional application: 06006494.6
(73) Proprietor: SCHERING CORPORATION, Kenilworth, New Jersey 07033-0530 (US)
(72) Inventor: TANG, John, Chu-Tay, Livingston, NJ 07039 (US); VELLEKAMP, Gary, J., Glen Ridge, NJ 07028 (US); BONDOC, Laureano, L., Jr., Piscataway, NJ 08854 (US)
(74) Representative: Naylor, Kathryn May
(86) International application number: PCT/US1997/022134
(87) International publication number: WO 1998/026048

(56) References cited:
- EP-A1- 0 522 291
- EP-A1- 0 593 339
- WO-A2-95/11984

## Description

### BACKGROUND TO THE INVENTION

The cultivation and purification of viruses has become increasingly important for gene therapy and vaccine development. Huyghe et al. (Human Gene Therapy 6: 1403-1416 (1995)) disclosed a comparison of several methods for purification of recombinant adenoviruses, including anion-exchange chromatography, size exclusion chromatography, immobilized zinc affinity chromatography, ultracentrifugation, concluding that the preferred process for purification of a recombinant adenovirus is nuclease treatment of a cell lysate, followed by filtration through membrane filters, followed by DEAE chromatography, followed by zinc affinity chromatography.

WO 97/08298 relates to a process for purifying adenovirus and adeno-associated virus.

WO 98/00524 relates to a method for producing recombinant adenovirus.

EP 0593339 A relates to methods of preparing Hepatitis A antigens and vaccines.

EP 0522291 A relates to a process for purifying Hepatitis A virus.

In view of the ever-increasing need for purified viruses, for example for use as viral vectors for gene therapy, improved methods of purification would be highly desired.

### SUMMARY OF THE INVENTION

One aspect of the invention is a method of purifying adenovirus from a virus preparation, comprising the successive steps of:
a) subjecting the virus preparation to anion-exchange chromatography, wherein the adenovirus is eluted from an anion-exchange chromatographic medium; and
b) subjecting the eluate of step a) which contains the adenovirus to size exclusion chromatography, wherein the adenovirus is eluted from a size exclusion chromatographic medium, wherein the size-exclusion medium is selected from the group consisting of G6000PWXL, SB-806, SEPHACRYL^{RTM} S-400 HR, SEPHADEX G-200, SEPHAROSE^{RTM} CL-2B. SUPERDEX^{RTM} 200 prep grade, SUPEROSE^{RTM} 6 prep grade, TSK 6000PWXL, and ULTRAHYDROGEL 2000. The virus preparation can be a cell lysate, which can be filtered before step a). The adenovirus can be recombinant adenovirus, such as ACN53 (disclosed in WO 95/11984).

The anion exchange medium can comprise diethylaminoethyl groups on a cross-linked agarose, cellulose, polyacrylamide or polystyrene backbone, such as FRACTOGEL^{™}-DEAE. The size-exclusion medium can comprise a cross-linked polysaccharide, and may be a composite of cross-linked agarose and dextran. An exemplary size exclusion medium is Superdex^{RTM}-200. The anion exchange chromatographic medium can be extensively washed before application of the virus preparation.

The size-exclusion medium can be provided in a column prepared as a salt gradient decreasing in ionic strength from the top of the medium towards the bottom, the top of the column having a buffer having an ionic strength substantially identical to that of the product of step a.

The invention also relates to a virus purified by the method of claim 1.

### DESCRIPTION OF THE PREFERRED EMBODIMENT

The present invention relates to the purification of a virus, namely adenovirus, which may for example have been produced by cultivation in a cellular host and then liberated by lysis of the cells and separation from cellular debris. The term "virus" includes wild type, mutant, and recombinant viruses, especially adenoviral vectors for expression of heterologous nucleic acid sequences.

The embodiments of the invention fall into the general strategy of adsorption chromatography of a virus preparation followed by size exclusion chromatography. Typically the anion exchange chromatography is carried out on an anion-exchange resin consisting of basic groups in side chains attached to a macromolecular backbone. The basic groups are preferably substituted amino groups, in particular diloweralkylaminoalkyl groups where each lower alkyl group has 1 to 4, preferably 2, carbon atoms, and each alkyl group has 2 to 4, preferably 2, carbon atoms. The backbone can be composed of silica or an organic matrix, for example cross-linked agarose, cellulose, polyacrylamide or polystyrene; it is particularly preferred to use an anion exchange resin consisting of dimethylaminoethyl groups (DMAE groups) or especially of diethylaminoethyl groups (DEAE groups) on a cross-linked agarose backbone; especially preferred resins of the DEAE type are those sold under the trade name "DEAE-Fractogel", e.g., "FRACTOGEL^{™} EMD DEAE-650M", and "FRACTOGEL^{™} AE. In some embodiments of the invention, the "backbone" can be a solid support such as a bead.

The anion-exchange-resin is preferably washed extensively before loading the virus preparation to remove preservatives such as sodium azide and ethanol, and other extraneous materials, by washing the column with about 5 to 10 column volumes of a basic solution such as 50 mM NaOH/1 M NaCl, followed by about 5 to 10 column volumes of a neutralizing solution such as 50 mM HCl/1 M NaCl, followed by about 5 to 30 volumes of loading and/or elution buffers. Optionally, the column is washed with a buffer of lower salt concentration than the loading and/or elution buffer before washing with loading and/or elution buffer.

Typically, a preparation of virus such as a cell lysate is loaded onto the chromatographic medium in a buffered solution of about pH 7.0 - 8.5, with a salt concentration of about 100-360 mM. The salt is typically NaCl. In some embodiments other buffers such as phosphate or Tris are used. Contaminants can be preferentially eluted by washing the column with a buffer at a salt concentration of about 250-380 mM. The adenovirus can then be eluted by a solution with a salt concentration of about 360-600 mM. The salt is typically NaCl. Typically, about 5 to 50, more preferably about 30 volumes of buffer are used to elute the virus. Fractions may be collected and assayed for the presence of virus by measuring the A₂₆₀ or A₂₈₀ and pooling peak fractions; alternatively, the eluant containing the A₂₆₀ or A₂₈₀ peak may be collected in a single fraction. This single A₂₆₀ or A₂₈₀ fraction or pooled fractions in the eluant containing the virus are termed "anion-exchange pool" herein.

In the size-exclusion chromatography step, molecules are separated according to size in a bed packed with an inert porous medium, especially an inert gel medium, which is preferably a composite of cross-linked polysaccharides, e.g., cross-linked agarose and dextran in the form of spherical beads. Molecules larger than the largest pores in the swollen gel beads do not enter the gel beads and therefore move through the chromatographic bed fastest. Smaller molecules, which enter the gel beads to varying extent depending on their size and shape, are retarded in their passage through the bed. Molecules are thus generally eluted in the order of decreasing molecular size. Viruses, because of their large size, generally elute in the void volume. For example, adenoviruses have a diameter of approximately 80 nm. Media appropriate for size-exclusion chromatography of adenoviruses the resins G6000PWXL (TosoHaas); SB-806 (Alltech); Sephacryl^{RTM}S-400 HR, Sephadex G-200, Sepharose^{RTM} CL-2B; Superdex^{RTM} 200 prep grade, Superose^{RTM} 6 prep grade (Pharmacia); TSK 6000PWXL (Bodman), and Ultrahydrogel 2000 (Waters).

"Size-exclusion" chromatography as used herein is intended to include gel filtration chromatography. A particularly preferred size-exclusion medium is that sold under the trade name "Superdex^{RTM} 200"; see the Pharmacia Catalog, 1996, pages 338-339, code no. 17-1043-01 (bulk), or 17-1069-01 or 17-1071-01 (pre-packed columns). Since a group separation of virus from impurities of lower molecular weight is achieved, the loading volume of starting materials from the anion-exchange pool can be relatively large, e.g., up to 20%, more preferably 15%, of the bed volume.

Exemplary materials for the practice of the anion-exchange and size exclusion chromatographic steps of the invention are provided in Table I. Exemplary variables and controls are provided in Tables II and III.

**Table I : Exemplary Materials Used In Anion-Exchange and Size Exclusion Chromatography**

| Purification Step | Procedure. | Solution Used |
|---|---|---|
| DEAE-Fractogel^{™} | Salt Adjustment | 4 M NaCl |
| | Equilibration | 265 mM NaCl, 2 mM MgCl₂, 2% (w/v) sucrose, 50 mM sodium phosphate at pH 7.5 (Buffer A) |
| | | 50 mM NaOH, 1M NaCl |
| | | 100 mM HCl, 1M NaCl |
| | Wash 1 | 265 mM NaCl, 2 mM MgCl₂, 2% (w/v) sucrose, 50 mM sodium phosphate at pH 7.5 (Buffer A) |
| | Wash 2 | 265 mM NaCl, 2 mM MgCl₂, 2% (w/v) sucrose, 50 mM sodium phosphate at pH 7.5 (Buffer A) |
| | | 600 mM NaCl, 2 mM MgCl₂, 2% (w/v) sucrose, 50 mM sodium phosphate at pH 7.5 (Buffer B) |
| | Elution | 265 mM NaCl, 2 mM MgCl₂, 2% (w/v) sucrose, 50 mM sodium phosphate at pH 7.5 (Buffer A) |
| | | 600 mM NaCl, 2 mM MgCl₂, 2% (w/v) sucrose, 50 mM sodium phosphate at pH 7.5 (Buffer B) |
| Superdex ^{RTM} 200 | Equilibration | 130 mM NaCl, 2 mM MgCl₂, 2% (w/v) sucrose, 50 mM sodium phosphate at pH 7.5 (Buffer C) |
| | Elution | 130 mM NaCl, 2 mM MgCl₂, 2% (w/v) sucrose 50 mM sodium phosphate at pH 7.5 (Buffer C) |

**Table II: In-process Control and Operating Variables for the Anion-Exchange Chromatography**

| Purification Procedure | | Variable | Recommended Value |
|---|---|---|---|
| All procedures | | Temperature | 4-12°C |
| Equilibration | | Flow rate | < 5 cm/min. |
| 1) NaOH/NaCl | | Volume | 5 column volumes |
| | | pH of effluent | 12.0 |
| 2) HCl/NaCl | | Volume | 6 column volumes |
| | | pH of effluent | 8.0 |
| 3) Buffer A | | Volume | 10 column volumes |
| | | pH of effluent | equivalent to Buffer A ±0.2 pH |
| Load | | Flow rate | <5 cm/min |
| | | Conductivity of the feed | 20-30 mS |
| Wash 1: | Buffer A | Flow rate | <5 cm/min |
| | | Volume | 4 column volumes |
| Wash 2: | Buffer A/ | Flow rate | <5 cm/min |
| Buffer B | | Volume | 8 column volumes |
| Elution: | Buffer A/ | Flow rate | < 5 cm/min |
| Buffer B | | Volume | 30 column volumes |
| Fraction Selection | | A₂₈₀ | >>background |

**Table III: In-Process Control and Operating Variables for the Size-Exclusion Chromatography**

| Purification Procedure | | Variable | Recommended Value |
|---|---|---|---|
| All procedures | | Temperature | 4-12°C |
| Equilibration: | | Flow rate | <1 cm/min |
| Buffer C | | Volume | 1 column volume |
| | | pH of Effluent | equivalent to Buffer C ±0.2 pH |
| Load | | Flow Rate | <1 cm/min |
| | | Volume | 0.2 column volumes |
| | | Concentration | 30 A₂₈₀/mL |
| Elution: | Buffer C: | Flow rate | <1 cm/min |
| | | Volume | 1 column volume |
| Fraction Selection | | A₂₈₀ | >>background |

In an embodiment of the invention, the virus is loaded from the anion-exchange pool onto a size-exclusion column. In some embodiments, the column is prepared with a salt gradient decreasing in ionic strength from the top towards the bottom of the column. After loading, the virus moves down through the salt gradient (since the virus is preferentially not adsorbed by the resin) and the gentle change in ionic strength avoids damage to the virus. After overtaking the salt gradient, the virus is eluted in a low-salt (e.g. 0-200 mM NaCl) buffer. Such low salt buffers include, but are not limited to, formulations for long term storage or administration to patients.

In some embodiments, glycerol is added to the chromatographic buffers, such as elution buffer, or to the pooled fractions containing virus. Typically the glycerol is present in a final concentration of 5-20%, more typically 10%. Thus, in some embodiments, glycerol is present in all solutions throughout the process. In further embodiments, other excipients, such as about 2-16% sucrose, may be used in place of the glycerol.

In a preferred embodiment, the size exclusion chromatography column is equilibrated with buffer at low salt concentration, e.g., about 100 to 150 mM, especially about 130 mM NaCl. Just prior to loading the feed, a salt gradient is loaded, equivalent to a moderate fraction of the bed volume, e.g. 10 to 20%, preferably about 15%, from low salt concentration (about 130 mM NaCl) to the higher salt concentration of the feed (e.g., 400 to 450 mM NaCl, especially about 420 mM NaCl).

A simple test is performed to determine the quality of the DEAE-Fractogel^{RTM} pool and consequently whether a salt gradient should be used. This test depends on the constancy of the A₃₂₀/A₂₆₀ ratio of the DEAE-Fractogel^{RTM} pool diluted with an appropriate pH 7.5 buffer over a period of a few minutes (e.g., 5 minutes). An appropriate buffer consists of 50 mM sodium phosphate, pH 7.5, 2 mM MgCl₂, 2% sucrose, no NaCl. If the A₃₂₀/A₂₆₀ ratio remains substantially constant over a 5-minute period (e.g., if it increases by no more than 0.04), then that sample is suitable for either isocratic or salt-gradient size-exclusion chromatography. If the ratio of A₃₂₀/A₂₆₀ increases more than about 0.04 during that period, then that DEAE-Fractogel pool is preferably performed on salt-gradient size-exclusion chromatography to improve the yield. Table IV provides exemplary materials and protocols for the use of salt gradient size exclusion chromatography.

**Table IV: Exemplary materials and protocols for the use of salt gradient size exclusion chromatography.**

| Step | Procedure | Typical Range | Preferred values |
|---|---|---|---|
| Step (I) | Equilibrate the column with the low salt buffer | 100 to 150 mM NaCl | 130 mM NaCl |
| Step (ii) | Generate salt gradient at top of column | (100-150) to (400-500) mM NaCl; | 130 to 450 mM NaCl; |
| | | 10 to 20% bed volume | 15% bed volume |
| Step (iii) | Load the DEAE-pool onto the column. | 10 to 20% bed volume | 15% bed volume |
| Step (iv) | Elute the adenovirus with high-salt solution | 400 to 500 mM NaCl | 450 mM NaCl |
| Step (v) | Complete the elution of the Virus. | 400 to 500 mM NaCl | 450 mM NaCl |

The purification method of the present invention is suitable for scaling-up (or scaling down) and for large-scale containment. Suitable procedures and guidelines well-known in the art can be used and followed to control the virus and prevent biohazardous situations: see, e.g., "Biosafety in Microbiological and Biomedical Laboratories", 3rd Edition, edited by Richman and McKinney, U.S. Department of Health and Human Services, published by the Center for Disease Control and the National Institute of Health, Washington, DC, U.S. Government Printing Office, May 1993.

The adenoviruses purified by the methods of the invention are preferably recombinant adenoviruses, but can include clinical isolates, attenuated vaccine strains, and so on.

Thus, for example, an exemplary recombinant adenovirus that can be purified by the method of the invention is ACN53, which is disclosed in PCT patent application no. WO 95/11984.

In the first step, the ACN53 adenoviral vector is purified by anion exchange chromatography on a DEAE-column. For this, the virus is typically propagated in 293 kidney cells, harvested, and subjected to concentration and ultrafiltration. The concentrate is frozen and stored at about -20°C until use. Frozen concentrate is thawed, clarified by filtration through a 0.45 µm filter, the conductivity of the preparation adjusted to about 250-360 mM NaCl, and subjected to DEAE chromatography. Solutions used in the chromatography are listed in Table 1.

In the second step, the ACN53 adenoviral vector is purified by size-exclusion chromatography on a Superdex^{RTM}-200 column. Selected fractions containing virus are identified by A₂₆₀ or A₂₈₀ and pooled. The pooled fractions constitute the purified bulk ACN53 adenoviral vector which is then filtered sterile through a 0.2 µm filter and stored at about -20°C.

The virus in the DEAE-Fractogel^{RTM} pool may be unstable due to the presence of a high concentration of salt (about 420 mM NaCl). It is preferably processed immediately or stored at 4-12°C for not more than about 24 hours.

Fractions of the elution profile showing the ACN53 adenoviral vector peak as determined by A₂₆₀ or A₂₈₀ are pooled for further processing. The size-exclusion pool is filtered through a 0.2 µm filter. This filtrate, the final purified bulk ACN53 adenoviral vector, is then transferred into sterile plastic bottles (e.g. Teflon) and stored at about -20°C. The in-process controls for this step are listed in Table III.

The increase in purity of the ACN53 adenoviral vector at each step of the purification method can be followed by Resource Q HPLC (see Huyghe et al., Human Gene Therapy, Vol. 6 (November 1995), pp. 1403-1416 at p. 1405). The quality of the virus in the first and second chromatographic pools is also monitored by spectroscopic methods. The characteristic ratio of A₂₆₀/A₂₈₀ is 1.23 -1.31:1 for final purified virus. The light scattering which results from the high molecular weight of the virus is derived from the ratio of A₃₂₀/A₂₆₀ nm and is also used to monitor the chromatographic pools. Purified, free virus particles display a light scattering ratio of about 0.22 -0.30:1.

The following Examples serve to illustrate the present invention. The selected vectors and hosts and other materials, the concentration of reagents, the temperatures, and the values of other variables are only to exemplify how the present invention may be carried out, and are not to be considered limitations thereof.

### EXPERIMENTAL EXAMPLES

### A. Small Scale Purification of Adenovirus

### (1) Anion-Exchange Chromatography (DEAE-Fractogel)

A DEAE-EMD Fractogel^{RTM} 650M column (E. Merck), 5 x 18 cm., was pre-equilibrated with 5 bed volumes (B.V.) of 0.5 M NaOH/ 1M NaCl followed by 6 B.V. of 0.1 M HCl/ 1M NaCl, and then by 20 B.V. of Buffer A (265 mM NaCl, 2 mM MgCl₂, 2% (w/v) sucrose, 50 mM sodium phosphate at pH 7.5) at a linear flow rate of 2 cm/min. The feed for this column was derived from 2 liters of frozen crude virus solution, which was thawed, microfiltered through a 0.45 µ membrane, and adjusted with a small volume of 4 M NaCl to a conductivity equal to that of Buffer A. The feed was loaded onto the column at a linear flow rate of 1 cm/min. The column was washed with 4 B.V. of Buffer A. The column was then washed with 8 B.V. of 94% Buffer A / 6% Buffer B (identical to Buffer A except that NaCl was 600 mM). The column was eluted with 30 B.V. of a linear gradient from 94% Buffer A / 6% Buffer B to 100% Buffer B. Fractions containing substantial virus were pooled to form the feed ("DEAE pool") for the following column.

### (2) Isocratic Size-Exclusion Chromatography (Superdex^{RTM}-200)

Size exclusion chromatography was performed on a Superdex^{RTM}-200 column (Pharmacia), 5 x 73 cm, pre-equilibrated with 0.5 B.V. 0.5 M NaOH, 1 B.V. of H₂O, and 2 B.V. of Buffer C (130 mM NaCl, 2 mM MgCl₂, 2% (w/v) sucrose, 50 mM sodium phosphate at pH 7.5) at a linear flow rate of 0.6 cm/min. The feed consisting of 220 ml of DEAE pool was loaded onto the column. ACN53 was eluted with Buffer C at a linear flow rate of 0.6 cm/min. Fractions with substantial virus were pooled, passed through a 0.2 µ microfilter and stored. The virus concentrate can be stored at low temperature, e.g., at 0-10°C, preferably at about 4°C; or if the volume is small, e.g., less than about 50 ml, frozen at -80°C.

### (3) Salt-Gradient Size-Exclusion Chromatography

### (a). Salt Dilution Test

The DEAE-Fractogel^{RTM} pool (0.4 ml) was mixed with of a buffer consisting of 50 mM sodium phosphate, pH 7.5, 2 mM MgCl₂, 2% sucrose, no NaCl (0.8 ml) and immediately placed in a quartz cuvette and measured for absorbance at 260 and 320 nm on a UV spectrometer equipped with a photodiode array. Without removal of the sample from the cuvette, the reading was repeated at 1-2 minute intervals over a 5-minute period. If the ratio of A₃₂₀/A₂₆₀ was substantially constant during that period, then that DEAE-Fractogel^{RTM} pool was suitable for either isocratic or salt-gradient size-exclusion chromatography. If the ratio of A₃₂₀/A₂₆₀ increased more than about 4% during that period, then that DEAE-Fractogel^{RTM} pool required salt-gradient size-exclusion chromatography to improve the yield.

### (b) Salt-Gradient Size-Exclusion Chromatography

A salt-gradient chromatography was performed on a Superdex^{RTM}-200 column, 2.6 cm x 60 cm, pre-equilibrated with 0.5 B.V. 0.5 M NaOH, 1 B.V. of H₂O, and 2 B.V. of Buffer C (20 mM sodium phosphate, pH 8.0, 130 mM NaCl, 2mM MgCl₂, 2%, sucrose). Immediately prior to loading the DEAE pool, a linear gradient from 100% Buffer C to 100% Buffer D (20 mM sodium phosphate, pH 8.0, 420 mM NaCl, 2 mM MgCl₂, 2% sucrose) of 0.15 B.V. (48 ml) was applied to the Superdex^{RTM}-200 column. The feed consisting of 20 ml of a DEAE pool that had failed the above test was then loaded onto the column and eluted with Buffer D at a linear flow rate of 0.6 cm/min. Fractions with substantial virus eluting in or near the void volume were pooled, passed through a sterilizing filter and stored at -80°C. The step yield was 60% and the A₃₂₀/A₂₆₀ ratio was 0.24:1.

### B. Large-Scale Purification of Adenovirus

### (1) Anion Exchange Chromatography

The frozen vial concentrate from the fermentation and recovery step was thawed and filtered through a 0.45 µm hydrophilic Durapore membrane in a Millipore 10" Opticap capsule. The filtrate was collected in a closed tank. To minimize losses, the filter cartridge was washed with about 1.5 L of buffer J-1 (50 mM sodium phosphate pH 7.5, 265 mM sodium chloride, 2 mM magnesium chloride, 2% (w/v) sucrose) supplemented with 5.4% (w/w) of solution J-3 (4 M sodium chloride). The salt concentration of the filtrate was adjusted by adding 5.4% (w/v) of solution J-3 (4 M sodium chloride). This feed solution was then applied to a Fractogel^{RTM} EMD DEAE-650 M column (7 cm diameter, 14.8 cm bed height, 570 ml bed volume) pre-equilibrated with buffer J-1 (50 mM sodium phosphate pH 7.5, 265 mM sodium chloride, 2 mM magnesium chloride, 2% (w/v) sucrose). The Adenovirus binds to the anion exchange resin, whereas the majority of media and host cell impurities pass through the column in the spent charge. The column was initially washed with 4 bed volumes of buffer J-1 followed by a second isocratic wash of 8 bed volumes of 94% buffer J-1 and 6% buffer J-2 (50 mM sodium phosphate pH 7.5, 600 mM sodium chloride, 2 mM magnesium chloride, 2% (w/v) sucrose) to remove additional impurities. The virus was eluted from the column with a 30 bed volume linear gradient from 6% to 100% buffer J-2. The adenovirus peak of the elution profile as determined by A₂₈₀ was collected and pooled for further processing. The in-process controls and operating parameters for the anion exchange chromatography step are summarized in Table V.

**Table V: In-Process Control and Operating Parameters for the Anion-Exchange Chromatography**

| Column size: 7 cm diameter, 14.8 cm bed height, 570 ml bed volume | | |
|---|---|---|
| Purification Procedure | Parameter | Value |
| All Procedures | Temperature | 3 to 9 °C |
| Equilibration | Flow Rate | 4 cm/min |
| | Volume | 22 Column Volumes |
| | pH of Effluent | Equal to Buffer J-1 (7.4) |
| Load | Flow Rate | 1 cm/min |
| | Conductivity of the Feed | 28.2 mS |
| Wash 1 | Flow Rate | 2 cm/min |
| | Volume | 4 Column Volumes. |
| Wash 2 | Flow Rate | 2 cm/min |
| | Volume | 8 Column Volumes |
| Elution | Flow Rate | 2 cm/min |
| | Volume | 30 Column Volumes |
| Fraction Selection | ^{A}280 | Peak area |

### (2) Size Exclusion Chromatography

The DEAE-Pool was applied immediately to a Superdex^{RTM}-200 size exclusion column (14 cm diameter, 77 cm bed height, 11.9 L bed volume) pre-equilibrated with buffer K-1 (20 mM sodium phosphate pH 8.0, 100 mM sodium chloride, 2 mM magnesium chloride, 2% (w/v) sucrose). The column was eluted with buffer K-1. The adenovirus peak of the elution profile as determined by A₂₈₀ was collected and pooled. This chromatography step achieved a buffer exchange and separation of low molecular weight impurities from the adenovirus product. The in-process controls and operating parameters for the size exclusion chromatography step are summarized in Table VI.

**Table VI: In-Process Control and Operating Parameters for the Size Exclusion Chromatography**

| Column size: 14 cm diameter, 77 cm bed height, 11.9 L bed volume | | |
|---|---|---|
| Purification Procedure | Parameter | Value |
| All Procedures | Temperature | 3 to 9 °C |
| Equilibration | Flow Rate | 0.43 cm/min |
| | Volume | 2.3 Column Volume |
| Load | Flow Rate | 0.43 cm/min |
| | Volume | ≤0.09 Column Volumes |
| | Concentration | 2.7 A₂₈₀/mL |
| Elution | Flow Rate | 0.43 cm.min |
| | Volume | 1.5 Column Volume |
| Fraction Selection | A₂₈₀ | Peak area |

### (3) Final 0.2 µm Filtration

The Superdex^{RTM}200-pool was filtered through a 0.2 µm hydrophilic Durapore membrane (Stericup, Millipore) at 2 to 15°C. This step was carried out under sterile conditions in a biosafety cabinet. Because several filtration devices were used, the individual filtrates were pooled and then aliquoted into autoclaved containers. The containers of bulk drug substance in solution were frozen in a dry ice/ethanol bath and stored at about -20°C.

Modifications and variations of this invention will be apparent to those skilled in the art. The specific embodiments described herein are offered by way of example only, and the invention is not to be construed as limited thereby.

## Claims

1. A method of purifying adenovirus from a virus preparation, comprising the successive steps of:
a) subjecting the virus preparation to anion-exchange chromatography, wherein the adenovirus is eluted from an anion-exchange chromatographic medium; and
b) subjecting the eluate of step a) which contains the adenovirus to size exclusion chromatography, wherein the adenovirus is eluted from a size exclusion chromatographic medium,
wherein the size-exclusion medium is selected from the group consisting of G6000PWXL, SB-806, SEPHACRYL S-400 HR, SEPHADEX G-200, SEPHAROSE CL-2B, SUPERDEX 200 prep grade, SUPEROSE 6 prep grade, TSK 6000PWXL, and ULTRAHYDROGEL 2000.

2. The method of claim 1, wherein the virus preparation is a cell lysate.

3. The method of claim 2, wherein the cell lysate is filtered before step a).

4. The method of claim 1, wherein the adenovirus is recombinant.

5. The method of claim 1 wherein the adenovirus is ACN53.

6. The method of claim 1 wherein the anion-exchange medium comprises dimethylaminoethyl groups or diethylaminoethyl groups on a cross-linked agarose, cellulose, polyacrylamide or polystyrene backbone.

7. The method of claim 6, wherein the anion-exchange medium is diethylaminoethyl -FRACTOGEL.

8. The method of claim 1, wherein the size-exclusion medium is SUPERDEX 200 prep grade.

9. The method of claim 1, wherein the anion-exchange chromatographic medium is extensively washed before step a).

10. The method of claim 1, wherein step b) further comprises eluting the adenovirus from the size-exclusion chromatographic medium in a low-salt buffer, wherein the size-exclusion medium is in a column containing a salt gradient which decreases in ionic strength from the top of the column towards the bottom of the column.

11. The method of claim 1, wherein the anion-exchange chromatographic medium or the size exclusion chromatographic medium is contacted with a buffer comprising glycerol or 2-16% sucrose.

12. The method of claim 11, wherein glycerol is present in all buffers used in the method.

13. The method of claim 1, further comprising a step of adding glycerol to a pooled fraction that contains the adenovirus.

## Patentansprüche

1. Verfahren zum Aufreinigen von Adenovirus aus einer Virusaufbereitung, das die folgenden aufeinanderfolgenden Schritte umfasst:
a) Unterwerfen der Virusaufbereitung einer Anionenaustauschchromatographie, wobei das Adenovirus aus einem Anionenaustauschchromatographie-Medium eluiert wird, und
b) Unterwerfen des Eluats aus Schritt a), welches das Adenovirus enthält, einer Größenausschlußchromatographie, wobei das Adenovirus aus einem Größenausschlußchromatographie-Medium eluiert wird,
wobei das Größenausschlußchromatographie-Medium ausgewählt ist aus der Gruppe bestehend aus G6000PWXL, SB-806, SEPHACRYL S-400 HR, SEPHADEX G-200, SEPHAROSE CL-2B, SUPERDEX 200 mit Präparationsgüte, SUPEROSE 6 mit Präparationsgüte, TSK 6000PWXL und ULTRAHYDROGEL 2000.

2. Verfahren gemäß Anspruch 1, bei dem die Virusaufbereitung ein Zelllysat ist.

3. Verfahren gemäß Anspruch 2, bei dem das Zelllysat vor Schritt a) gefiltert wird.

4. Verfahren gemäß Anspruch 1, bei dem das Adenovirus rekombinant ist.

5. Verfahren gemäß Anspruch 1, bei dem das Adenovirus ACN53 ist.

6. Verfahren gemäß Anspruch 1, bei dem das Anionenaustausch-Medium Dimethylaminoethylgruppen oder Diethylaminoethylgruppen auf einem kreuzvernetztem Agarose-, Cellulose-, Polyacrylamid- oder Polystyrengrundgerüst umfasst.

7. Verfahren gemäß Anspruch 6, bei dem das Anionenaustausch-Medium Diethylaminoethyl-FRACTOGEL ist.

8. Verfahren gemäß Anspruch 1, bei dem das Größenausschluß-Medium SUPERDEX 200 mit Präparationsgüte ist.

9. Verfahren gemäß Anspruch 1, bei dem das Anionenaustauschchromatographie-Medium vor Schritt a) gründlich gewaschen wird.

10. Verfahren gemäß Anspruch 1, bei dem Schritt b) weiterhin das Eluieren des Adenovirus aus dem Größenausschlußchromatographie-Medium in einen Niedrigsalz-Puffer umfasst, wobei das Größenausschluß-Medium in einer Säule vorliegt, die einen Salzgradienten aufweist, dessen Ionenstärke von der Spitze der Säule zum Boden der Säule hin abnimmt.

11. Verfahren gemäß Anspruch 1, bei dem das Anionenaustauschchromatographie-Medium oder das Größenausschlußchromatographie-Medium mit einem Puffer in Kontakt gebracht wird, der Glycerin oder 2-16 % Saccharose umfasst.

12. Verfahren gemäß Anspruch 11, bei dem Glycerin in allen Puffern vorliegt, die in dem Verfahren verwendet werden.

13. Verfahren gemäß Anspruch 1, das ferner einen Schritt des Zugebens von Glycerin in eine vereinte Fraktion umfasst, welche das Adenovirus enthält.

## Revendications

1. Procédé de purification d'un adénovirus à partir d'une préparation de virus, qui comporte les étapes successives indiquées ci-dessous :
a) soumettre la préparation de virus à une chromatographie d'échange d'anions, opération au cours de laquelle l'adénovirus est élué d'un milieu de chromatographie d'échange d'anions,
b) et soumettre l'éluat issu de l'étape (a), qui contient l'adénovirus, à une chromatographie d'exclusion stérique, opération au cours de laquelle l'adénovirus est élué d'un milieu de chromatographie d'exclusion stérique,
lequel milieu de chromatographie d'exclusion stérique est choisi dans l'ensemble formé par les G6000PWXL, SB-806, Séphacryl S-400HR, Séphadex G-200, Sépharose CL-2B, Superdex 200 de qualité préparative, Superose 6 de qualité préparative, TSK 6000PWXL, et Ultrahydrogel 2000.

2. Procédé conforme à la revendication 1, dans lequel la préparation de virus est un lysat de cellules.

3. Procédé conforme à la revendication 2, dans lequel le lysat de cellules est filtré avant l'étape (a).

4. Procédé conforme à la revendication 1, dans lequel l'adénovirus est recombiné.

5. Procédé conforme à la revendication 1, dans lequel l'adénovirus est ACN53.

6. Procédé conforme à la revendication 1, dans lequel le milieu de chromatographie d'échange d'anions comporte des groupes diméthylaminoéthyle ou diéthylaminoéthyle, attachés à un squelette réticulé d'agarose, de cellulose, de polyacrylamide ou de polystyrène.

7. Procédé conforme à la revendication 6, dans lequel le milieu de chromatographie d'échange d'anions est du diéthylaminoéthyl-Fractogel.

8. Procédé conforme à la revendication 1, dans lequel le milieu de chromatographie d'exclusion stérique est du Superdex 200 de qualité préparative.

9. Procédé conforme à la revendication 1, dans lequel le milieu de chromatographie d'échange d'anions subit un lavage poussé, avant l'étape (a).

10. Procédé conforme à la revendication 1, dans lequel l'étape (b) comporte en outre le fait d'éluer l'adénovirus du milieu de chromatographie d'exclusion stérique dans une solution tampon à faible teneur en sel, alors que ledit milieu de chromatographie d'exclusion stérique se trouve dans une colonne à gradient de sel où la force ionique diminue de haut en bas de la colonne.

11. Procédé conforme à la revendication 1, dans lequel le milieu de chromatographie d'échange d'anions ou le milieu de chromatographie d'exclusion stérique est mis en contact avec une solution tampon contenant du glycérol ou 2 à 16 % de saccharose.

12. Procédé conforme à la revendication 11, dans lequel, dans toutes les solutions tampons employées, il y a du glycérol.

13. Procédé conforme à la revendication 1, qui comporte en outre une étape où l'on ajoute du glycérol à un regroupement de fractions contenant l'adénovirus.
